# EUROPEAN PATENT SPECIFICATION

(11) **EP 4 414 361 B1**
(45) Date of publication and mention of the grant of the patent: **11.11.2026**
(21) Application number: 22901698.5
(22) Date of filing: 28.11.2022
(51) Int. Cl.: C07C 263/10, C07C 263/20, C07C 265/14

(54) **METHOD FOR PREPARING DIISOCYANATE**
VERFAHREN ZUR HERSTELLUNG VON DIISOCYANAT
PROCÉDÉ DE PRODUCTION DE DIISOCYANATE

(30) Priority: 30.11.2021 KR 20210169321
(43) Date of publication of application: 14.08.2024
(73) Proprietor: Hanwha Solutions Corporation, Jung-gu Seoul 04541 (KR)
(72) Inventor: PARK, Jongseong, Daejeon 34128 (KR); SIM, Yujin, Daejeon 34128 (KR); PARK, Juyoung, Daejeon 34128 (KR); WOO, Eun Ji, Daejeon 34128 (KR)
(74) Representative: Berggren Oy
(86) International application number: PCT/KR2022/018934
(87) International publication number: WO 2023/101340

(56) References cited:
- CN-A- 112 920 372
- JP-A- H05 163 231
- KR-A- 20050 089 053
- KR-A- 20190 139 153
- KR-A- 20210 071 802
- KR-B1- 102 217 747
- US-A- 5 962 728

## Description

### [Technical Field]

### Cross-reference to Related Application

This application claims the benefit of Korean Patent Application No. 10-2021-0169321 filed on November 30, 2021 with the Korean Intellectual Property Office.

The present disclosure relates to a method for preparing diisocyanate having high purity and improved stability.

### [Background Art]

An isocyanate compound is a compound highly useful as fine chemical products including optical materials, as well as in the fields of chemical industry and resin industry. Xylylene diisocyanate(hereinafter referred to as XDI), which is a representative example of isocyanate compounds, is raw material of high-quality optical lens and high value chemical material, and there is an increasing demand therefor.

Such isocyanate has high reactivity, and thus, is prone to degeneration according to storage environment. In case isocyanate is degenerated, not only purity is lowered, but also transparency is lowered and whitening occurs, and thus, it is unsuitable for use in the field of optics requiring excellent appearance property, particularly transparency.

Thus, a method of prescribing a stabilizer for isocyanate is being used, but the stabilizer may cause coloration, and in case stability of isocyanate itself is lowered, the effect is limited.

Accordingly, there is a demand for studies on the preparation of isocyanate in which whitening is inhibited, purity is maintained, and there is no concern about degeneration, even during long-term storage. CN 112 920 372 discloses a process for the preparation of diisocyanate from phosgene and the corresponding diamine followed by distillation at temperatures between 100 to 130°C. In example 1-1 reaction of m-XDA at 125°C is described. Purification is carried out by distillation at 60°C for 4 to 8 hours and at 120°C for 10 hours. In example 3-1 purification is carried out at 0.5 Torr (0.06 kPa) at 60°C for 8 hours and at 100°C for 8 hours.

### [Disclosure]

### [Technical Problem]

It is an object of the invention to provide a method for preparing diisocyanate that has high purity, and has excellent stability even during long-term storage, and thus, is suitable for the preparation of optical resin.

### [Technical Solution]

According to one embodiment of the invention, there is provided a method for preparing diisocyanate comprising:
a reaction step in which diamine or a salt thereof is reacted with phosgene to obtain a reaction mixture; and
a purification step in which diisocyanate is separated from the reaction mixture,
wherein the purification step is conducted at a temperature less than 170 °C for 16 hours or less, wherein the purification step comprises steps of: conducting vacuum distillation of the reaction mixture under a first temperature and a first pressure to remove solvents; conducting vacuum distillation under a second temperature and a second pressure to remove low-boiling impurities; and conducting thin film distillation under a third temperature and a third pressure to remove oligomers and wherein the third temperature is equal to or lower than the first temperature and/or the second temperature, and the third pressure is equal to or lower than the first pressure and/or the second pressure.

The diamine may be one or more selected from the group consisting of 1,2-xylylene diamine, 1,3-xylylene diamine, 1,4-xylylene diamine, 1,2-bis(aminomethyl)cyclohexane, 1,3-bis(aminomethyl)cyclohexane, and 1,4-bis(aminomethyl)cyclohexane, and
the diamine salt may be one or more selected from the group consisting of 1,2-xylylene diamine hydrochloride, 1,3-xylylene diamine hydrochloride, 1,4-xylylene diamine hydrochloride, 1,2-bis(aminomethyl)cyclohexane hydrochloride, 1,3-bis(aminomethyl)cyclohexane hydrochloride, 1,4-bis(aminomethyl)cyclohexane hydrochloride, 1,2-xylylene diamine carbonate, 1,3-xylylene diamine carbonate, 1,4-xylylene diamine carbonate, 1,2-bis(aminomethyl)cyclohexane carbonate, 1,3-bis(aminomethyl)cyclohexane carbonate, and 1,4-bis(aminomethyl)cyclohexane carbonate.

According to one embodiment, the reaction step may be conducted at a temperature of 80 °C to 180 °C.

According to one embodiment, the purification step may be conducted at a temperature of 100 °C to 150 °C for 5 hours to 15 hours.

According to one embodiment, the purification step may be conducted under pressure of 0.001 to 50 kPa by vacuum distillation and/or thin film distillation.

The purification step comprises steps of: conducting vacuum distillation of the reaction mixture under a first temperature and a first pressure to remove solvents; conducting vacuum distillation under a second temperature and a second pressure to remove low-boiling impurities; and conducting thin film distillation under a third temperature and a third pressure to remove oligomers. Wherein, the third temperature is equal to or lower than the first temperature and/or the second temperature, and the third pressure is equal to or lower than the first pressure and/or the second pressure.

After the purification step, the purity of diisocyanate may be 99 % to 100 %.

### [Effect of the Invention]

According to the preparation method of diisocyanate of the invention, diisocyanate that has high purity and has excellent stability even during long-term storage may be provided.

### [Best Mode for Carrying Out the Invention]

The terms used herein are only to explain specific embodiments, and are not intended to limit the invention. A singular expression includes a plural expression thereof, unless it is expressly stated or obvious from the context that such is not intended. As used herein, the terms "comprise" , "equipped" or "have" , etc. are intended to designate the existence of practiced characteristic, number, step, constructional element or combinations thereof, and they are not intended to preclude the possibility of existence or addition of one or more other characteristics, numbers, steps, constructional elements or combinations thereof.

Although various modifications can be made to the invention and the invention may have various forms, specific examples will be illustrated and explained in detail below. However, it should be understood that these are not intended to limit the invention to specific disclosure

Hereinafter, the invention will be explained in detail.

The inventors have studied on the preparation method of diisocyanate that can not only increase purity, but also improve stability so as not to generate degeneration such as whitening during long-term storage, and as the result, confirmed that the above effects can be achieved by controlling the highest temperature and residence time in the purification step, and completed the invention.

Thus, according to one embodiment of the invention, there is provided a method for preparing diisocyanate comprising:
a reaction step in which diamine or a salt thereof is reacted with phosgene to obtain a reaction mixture; and
a purification step in which diisocyanate is separated from the reaction mixture,
wherein the purification step is conducted at a temperature less than 170 °C for 16 hours or less.

Hereinafter, the invention will be explained according to steps.

First, a reaction step (phosgenation reaction step) in which diamine or a salt thereof is reacted with phosgene to obtain a reaction mixture, is conducted.

The diamine is aromatic, alicyclic, or aliphatic amine comprising two amine groups in the molecule. According to one embodiment, the diamine is one or more selected from the group consisting of 1,2-xylylene diamine(o-xylylene diamine, o-XDA), 1,3-xylylene diamine(m-xylylene diamine, m-XDA), 1,4-xylylene diamine(p-xylylene diamine, p-XDA), 1,2-bis(aminomethyl)cyclohexane, 1,3-bis(aminomethyl)cyclohexane, and 1,4-bis(aminomethyl)cyclohexane, and it may be selected according to aimed structure of diisocyanate.

The salt of diamine refers to a salt produced by the reaction of the diamine with acid, and for example, it may be hydrochloride prepared by the reaction of diamine and anhydrous hydrochloric acid, carbonate prepared by the reaction of diamine and carbonic acid. Although diamine rapidly reacts with phosgene, in case it is converted into a solid-state salt and used, the reaction speed may be mitigated.

Specifically, as the diamine salt, one or more selected from the group consisting of 1,2-xylylene diamine hydrochloride, 1,3-xylylene diamine hydrochloride, 1,4-xylylene diamine hydrochloride, 1,2-bis(aminomethyl)cyclohexane hydrochloride, 1,3-bis(aminomethyl)cyclohexane hydrochloride, 1,4-bis(aminomethyl)cyclohexane hydrochloride, 1,2-xylylene diamine carbonate, 1,3-xylylene diamine carbonate, 1,4-xylylene diamine carbonate, 1,2-bis(aminomethyl)cyclohexane carbonate, 1,3-bis(aminomethyl)cyclohexane carbonate, and 1,4-bis(aminomethyl)cyclohexane carbonate may be used.

The preparation of the diamine salt may be conducted in a solvent, and as the solvent, aromatic hydrocarbon solvents such as benzene, toluene, xylene, ethylbenzene, and the like; chlorinated aromatic hydrocarbon solvent such as monochlorobenzene, 1,2-dichlorobenzene, 1,4-dichlorobenzene, and the like; chlorinated hydrocarbon solvent such as dichloromethane, chloroform, carbon tetrachloride, and the like, may be used, and two or more kinds thereof may be used in combination. Since these solvents may be also used as the solvent of the phosgenation reaction, after obtaining diamine salt by reacting diamine and acid in the above-described solvent, without a separate purification step, phosgene may be introduced to conduct a phosgenation reaction.

It is preferable that the preparation of the diamine salt may be conducted at a temperature of 60 °C or less, preferably about 5 to 30 °C, and the temperature may be temporarily increased due to reaction heat during the reaction, but it is preferable that the highest temperature in the reactor is maintained at 90 °C or less, or 60 °C or less.

Meanwhile, in case diamine is reacted with phosgene, the phosgene may be introduced batchwise at the beginning of the reaction, or a part of the phosgene may be introduced at the beginning of the reaction, and then, the remainder may be introduced during the reaction. When introducing the phosgene, it is preferable that the temperature of the phosgene may be -10 °C to 0 °C because leakage of highly toxic phosgene may be prevented and the temperature of the reaction may be smoothly increased.

The temperature of the reactor during the phosgenation reaction may be controlled to 80 °C to 180 °C. Preferably, it may be 100 °C or more, or 120 °C or more, and 150 °C or less, or 140 °C or less. Under such temperature conditions, the phosgenation reaction may be smoothly progressed, and thermal decomposition of prepared diisocyanate may be prevented.

As additives for inhibiting the generation of by-products during the reaction of diamine and phosgene, one or more selected from the group consisting of 2,2,6,6-tetramethylpiperidine-1-oxyl(TEMPO), 4-hydroxy-2,2,6,6-tetramethylpiperidin-1-oxyl(hereinafter, referred to as 4-hydroxy TEMPO), 4-methoxy-2,2,6,6-tetramethylpiperidine 1-oxyl(4-methoxy-TEMPO), 2,2,6,6-tetramethyl-4-benzyl oxypiperidine-1-oxyl(4-benzyloxy-TEMPO), and 4-acetamido-2,2,6,6-tetramethylpiperidin-1-oxyl(hereinafter referred to as 4-acetamido TEMPO) may be used. In case the additive is used, the content of the additive may be 0.01 to 10 parts by weight, preferably 0.1 to 5 parts by weight, or 0.5 to 3 parts by weight, based on 100 parts by weight of the diamine or diamine salt.

Next, a purification step in which diisocyanate is separated from the reaction mixture obtained after the phosgenation reaction, is conducted.

Since isocyanate groups are highly reactive and prone to generate side-reactions, and impurities formed through the side-reactions may have an influence on the purity, chromacity, and the like of an isocyanate compound, a purification step is required. However, as the result of the studies of the inventors, it was confirmed that in case the purification step is conducted under excessively high temperature condition, or a time of the purification step increases, the stability of the isocyanate compound may be significantly deteriorated. Thus, in the present disclosure, the stability of diisocyanate may be improved by maintaining the temperature of the purification step at an optimum level, and controlling the total time(residence time) of the purification step.

Specifically, in the preparation method of the invention, the purification step is conducted at a temperature less than 170 °C for 16 hours or less. If the highest temperature of the purification step is 170 °C or more, or the residence time in the purification step is greater than 16 hours, prepared diisocyanate may absorb excessive heat, and thus, stability may be lowered. However, in case the temperature and residence time of the purification step are respectively controlled to less than 170 °C and 16 hours or less, the effect of improving stability of diisocyanate may be secured regardless of the amount of the reaction mixture to be purified.

Preferably, the highest temperature of the purification step is maintained at 160 °C or less, or 150 °C or less. Meanwhile, since the lowest temperature of the purification step does not have a significant influence on the stability of diisocyanate, it is not particularly limited, but considering the efficiency of the purification process, it may be 100 °C or more, or 110 °C or more.

And, the residence time in the purification step may be 16 hours or less, or 15 hours or less. As the residence time in the purification step is shorter, the stability of diisocyanate may be improved, but since a purification process for a certain time is required for purification with high purity, the purification time is preferably 5 hours or more, or 8 hours or more, or 10 hours or more, or 13 hours or more.

According to one embodiment, the purification step conducted by thin film distillation and vacuum distillation may use a distillation tower. The vacuum distillation and/or thin film distillation may be conducted under pressure of 0.01 to 50 kPa so as to meet the above explained temperature and residence time and enable purification with high purity.

As the distillation tower used for vacuum distillation, a plate tower, or a packed tower may be used without limitations. The theoretical number of stages of the distillation tower may be, for example, 2 or more, or 5 or more, or 10 or more, and 60 or less, or 40 or less.

A temperature of vacuum distillation is maintained less than 170 °C, and specifically, it may be 100 °C or more, or 130 °C or more, and 160 °C or less, or 150 °C or less. A pressure of vacuum distillation may be 0.001 kPa or more, or 0.005 kPa or more, or 0.01 kPa or more, and 50 kPa or less, 30 kPa or less, 10 kPa or less, or 1 kPa or less. The temperature and pressure of vacuum distillation mean the temperature and pressure of the bottom of the distillation tower.

And, the temperature of the top of the distillation tower during vacuum distillation may be 10 °C or more, or 20 °C or more, and 100 °C or less, or 80 °C or less, and the pressure may be 0.001 kPa or more, or 0.005 kPa or more, or 0.01 kPa or more, and 50 kPa or less, 30 kPa or less, 10 kPa or less, or 1 kPa or less, like the pressure of the bottom.

The thin film distillation may be conduced using a thin film evaporator equipped with an evaporator, a condenser, and a decompression means. The rotation speed of the rotator of the thin film evaporator may be 50rpm or more, or 120rpm or more, or 200rpm or more, and 500rpm or less, 400rpm or less, or 350rpm or less. A temperature of thin film distillation is also maintained less than 170 °C, and for example, it may be 100 °C or more, or 110 °C or more, and 160 °C or less, 130 °C or less, and the pressure may be 0.001 kPa or more, or 0.005 kPa or more, or 0.01 kPa or more, and 50 kPa or less, 30 kPa or less, 10 kPa or less, or 1 kPa or less.

Under these vacuum distillation conditions and/or thin film distillation conditions, purification efficiency may be increased while reducing residence time in the purification time, thereby obtaining a diisocyanate compound with high purity.

According to one embodiment, the purification step may be conducted in multi-stages while changing temperature and/or pressure conditions. For example, the purification step may be conducted by sequentially progressing solvent removal, low-boiling impurities removal, and oligomer removal steps.

Specifically, the purification step may be conducted by progressing vacuum distillation for removing solvents under a first temperature and a first pressure, and subsequently, removing low-boiling impurities under a second temperature and a second pressure, and then, removing oligomers under a third temperature and a third pressure, provided that it may be conducted by under a temperature less than 170 °C and a pressure of 0.001 to 50 kPa; or a temperature of 130 to 150 °C and a pressure of 0.01 to 1 kPa.

Wherein, the first temperature to third temperature may be identical to or different from each other, and the first pressure to third pressure may be identical to or different from each other. For example, the third temperature may be equal to or lower than the first temperature and/or second temperature, and the third pressure may be equal to or lower than the first pressure and/or second pressure.

Meanwhile, before the vacuum distillation step, a nitrogen bubbling step for removing unreacted phosgene and hydrogen chloride gas inside the reactor after the phosphgenation reaction is completed, may be further conducted.

By the above-explained preparation method, diisocyanate may be prepared. The diisocyanate may be one or more selected from the group consisting of 1,2-xylylene diisocyanate, 1,3-xylylene diisocyanate, 1,4-xylylene diisocyanate, 1,2-bis(isocyanatomethyl)cyclohexane, 1,3-bis(isocyanatomethyl)cyclohexane, and 1,4-bis(isocyanatomethyl)cyclohexane, according to diamine used.

The diisocyanate prepared according to the preparation method of the invention may have purity of 99% to 100% after purification. Preferably, the purity of diisocyanate may be 99.2% or more, or 99.4% or more, more preferably, 100%.

Meanwhile, since the diisocyanate is prepared through the purification step in which a temperature and residence time are controlled as explained above, it has excellent stability, and thus, little deterioration even during long-term storage, and has excellent color and transparency even during long-term storage.

Specifically, a difference between the purify of the diisocyanate when stored under refrigeration condition(4 °C) for 6 months after preparation, and the purity immediately after preparation may be 1.5% or less, or 1% or less.

For example, when storing the diisocyanate under refrigeration condition(4 °C) for 6 months after preparation, it may have purify of 98% or more, or 98.4% or more, or 98.6% or more. The higher the purity after storage, the more excellent it is, and it may be theoretically 100%, but for example, may be 99.5% or less, or 99% or less.

And, when storing the diisocyanate under refrigeration condition(4 °C) for 6 months after preparation, the content of white substances may be less than 0.8 wt%, 0.5 wt% or less, 0.3 wt% or less, or 0.1 wt% or less, and preferably 0 wt%.

Measurement methods of the purity, purity after refrigeration storage and white residue content of the diisocyanate will be embodied in examples described below.

The diisocyanate prepared by the above explained preparation method of the invention has excellent stability and transparency, and thus, may be suitably used for a polymeric composition for preparing optical devices. The polymeric composition may comprise, for example, the diisocyanate of the invention, and polyol and/or polythiol components.

As such, since the polymeric composition has excellent transparency and little yellowing, it may be suitably used for optical material for preparing glass lens, camera lens, and the like.

Hereinafter, preferable examples are presented for better understanding of the invention, but the following examples are presented only as the illustrations of the invention, and it is obvious to one of ordinary knowledge in the art that various modifications and alterations can be made

### [Example]

### Example 1

### (1) Reaction step

In a reactor, 471 g of 1,2-dichlorobenzene, 32.5 g of meta- xylylene diamine(m-XDA) with a purity of 99.4%, and 0.24 g of 4-hydroxy TEMPO were put, and stirred while introducing anhydrous hydrochloric acid at a speed of 20 g/hr at room temperature(23±5°C). During the process of introducing anhydrous hydrochloric acid, the temperature increased to 50°C.

After introducing anhydrous hydrochloric acid for 4 hours, salts formed were cooled to a room temperature, and 43 g of phosgene was introduced in the reactor, and then, the reactor was heated to a temperature of 130°C. Wherein, phosgene was prevented from being discharged outside with a dry ice-acetone cooling device from the time when phosgene was introduced till the time when the reaction was finished.

After the temperature of the reactor reached 130 °C, the temperature of the reactor was maintained as 125-135 °C for 2 hours such that the reaction solution became transparent. The end of the reaction was confirmed by transparentization of the solution.

### (2) Purification step

After the step (1) was completed, while blowing nitrogen inside the reactor, it was cooled to a room temperature. The phosgene-removed reaction mixture was subjected to vacuum distillation using 20-stage plate tower under conditions of pressure of 10.0 kPa, bottom temperature of 150 °C, and top temperature of 60 °C, thus removing solvents, and subsequently, subjected to vacuum distillation under conditions of pressure of 1.0 kPa, bottom temperature of 150 °C, and top temperature of 60 °C, thus removing low-boiling impurities, and then, evaporated at 130 °C, 0.5 kPa using a thin film evaporator equipped with a rotator rotating at 200rpm, thus removing oligomers, thereby obtaining meta-xylylene diisocyanate(m-XDI).

The highest temperature in the purification step was 150°C, which was the temperature of vacuum distillation, and the purification step took total 15 hours.

### Example 2

The phosgenation reaction of m-XDA was conducted by the same method as the step (1) of Example 1.

And then, while blowing nitrogen inside the reactor, it was cooled to a room temperature. The phosgene-removed reaction mixture was subjected to vacuum distillation using 20-stage plate tower under conditions of pressure of 5.0 kPa, bottom temperature of 140 °C, and top temperature of 50 °C, thus removing solvents, and subsequently, subjected to vacuum distillation under conditions of pressure of 0.5 kPa, bottom temperature of 140 °C, and top temperature of 50 °C, thus removing low-boiling impurities, and then, evaporated at 130 °C, 0.5 kPa using a thin film evaporator equipped with a rotator rotating at 200rpm, thus removing oligomers, thereby obtaining m-XDI.

The highest temperature in the purification step was 140°C, which was the temperature of vacuum distillation, and the purification step took total 16 hours.

### Comparative Example 1

The phosgenation reaction of m-XDA was conducted by the same method as the step (1) of Example 1.

And then, while blowing nitrogen inside the reactor, it was cooled to a room temperature. The phosgene-removed reaction mixture was subjected to vacuum distillation using 20-stage plate tower under conditions of pressure of 5.0 kPa, bottom temperature of 140 °C, and top temperature of 50 °C, thus removing solvents, and subsequently, subjected to vacuum distillation under conditions of pressure of 0.5 kPa, bottom temperature of 140 °C, and top temperature of 50 °C, thus removing low-boiling impurities, and then, evaporated at 130 °C, 0.5 kPa using a thin film evaporator equipped with a rotator rotating at 200rpm, thus removing oligomers, thereby obtaining m-XDI.

The highest temperature in the purification step was 140°C, which was the temperature of vacuum distillation, and the purification step took total 32 hours.

### Comparative Example 2

The phosgenation reaction of m-XDA was conducted by the same method as the step (1) of Example 1.

And then, while blowing nitrogen inside the reactor, it was cooled to a room temperature. The phosgene-removed reaction mixture was subjected to vacuum distillation using 20-stage plate tower under conditions of pressure of 10.0 kPa, bottom temperature of 150 °C, and top temperature of 60 °C, thus removing solvents, and subsequently, subjected to vacuum distillation under conditions of pressure of 1.0 kPa, bottom temperature of 150 °C, and top temperature of 60 °C, thus removing low-boiling impurities, and then, evaporated at 130 °C, 0.5 kPa using a thin film evaporator equipped with a rotator rotating at 200rpm, thus removing oligomers, thereby obtaining m-XDI.

The highest temperature in the purification step was 150°C, which was the temperature of vacuum distillation, and the purification step took total 24 hours.

### Comparative Example 3

The phosgenation reaction of m-XDA was conducted by the same method as the step (1) of Example 1.

And then, while blowing nitrogen inside the reactor, it was cooled to a room temperature. The phosgene-removed reaction mixture was subjected to vacuum distillation using 20-stage plate tower under conditions of pressure of 10.0 kPa, bottom temperature of 150 °C, and top temperature of 60 °C, thus removing solvents, and subsequently, subjected to vacuum distillation under conditions of pressure of 1.0 kPa, bottom temperature of 150 °C, and top temperature of 60 °C, thus removing low-boiling impurities, and then, evaporated at 0.5 kPa using a thin film evaporator equipped with a rotator rotating at 200rpm, thus removing oligomers, thereby obtaining m-XDI.

The highest temperature in the purification step was 150°C, which was the temperature of vacuum distillation, and the purification step took total 32 hours.

### Comparative Example 4

The phosgenation reaction of m-XDA was conducted by the same method as the step (1) of Example 1.

And then, while blowing nitrogen inside the reactor, it was cooled to a room temperature. The phosgene-removed reaction mixture was subjected to vacuum distillation using 20-stage plate tower under conditions of pressure of 50.0 kPa, bottom temperature of 170 °C, and top temperature of 80 °C, thus removing solvents, and subsequently, subjected to vacuum distillation under conditions of pressure of 10.0 kPa, bottom temperature of 170 °C, and top temperature of 80 °C, thus removing low-boiling impurities, and then, evaporated at 0.5 kPa using a thin film evaporator equipped with a rotator rotating at 200rpm, thus removing oligomers, thereby obtaining m-XDI.

The highest temperature in the purification step was 170°C, which was the temperature of vacuum distillation, and the purification step took total 16 hours.

### Experimental Examples

### (1) Measurement of XDI purity

The purity of each m-XDI of Examples and Comparative Examples was analyzed by GC. First, GC analysis was conducted for the m-XDI composition immediately after purification, and after storing the m-XDI under refrigeration(4 °C) condition for 6 months, GC analysis was conducted. For m-XDI in which whitening progressed after 6 months, insoluble white substances were filtered, and then, GC analysis was conducted for the filtrate.

GC used for analysis was HP-6890, and FID was used for detection. The column used was DB-17(30m * 0.25mm * 0.5*µ*m), carrier gas was nitrogen(1.0 mL/min), and the oven temperature was 80 °C -> 5 °C/min -> 160 °C(8 min) -> 20 °C/min -> 280 °C(18min).

### (2) Evaluation of degree of whitening and measurement of content of white substances

After storing m-XDI under refrigeration(4 °C) condition for 6 months, it was confirmed with the naked eye whether or not whitening occurred. 300 g of m-XDI in which whitening occurred, when observed with the naked eye, was weighed and filtered with a filter, and the weight of the filtered solid(white substances) was measured, and the content of white substances(wt%) was calculated therefrom.

### (3) Preparation of optical material and evaluation of transparency

Using each m-XDI obtained in Examples and Comparative Examples, a polymeric composition and an optical device(plastic lens) were prepared as follows.

20.8 g of m-XDI, 0.04 g of zelec UN(release agent, acidic phosphate ester, produced by Stepan Company), and 0.04 g of biosorb 583(UV absorber, 2-(2' -hydroxy-5' -t-octylphenyl) benzotriazole, produced by Sakai Chemical industry Co., Ltd) were stirred in a flask of room temperature for about 20 minutes.

After confirming with the naked eye that all the components were properly mixed, 0.002 g of dibutyltin chloride was additionally put and stirred for 10 minutes to form a mixture. To the mixture, 19.2 g of 2,3-bis(2-sulfanyl ethyl sulfanyl)propane-1-thiol was added, and stirred for 1 hour while defoaming at 5 mbar, thus preparing a polymeric composition.

The polymeric composition was filtered through 1 µm PTFE filter, and then, introduced into a mold consisting of a glass mold and a tape. The mold was introduced in an oven, and while gradually raising the temperature from 10 °C to 120 °C, polymerization was conducted for 20 hours. After the polymerization was finished, the mold was taken from the oven and released, followed by annealing at 120 °C for 6 hours to prepare plastic lens.

For the lens, the degree of whitening was evaluated with the naked eye using a common fluorescent lamp and a zirconium lamp(Y-100G).

### <Evaluation standard>

C(Clear): transparent both under fluorescent lamp and a zirconium lamp
S.H(Slightly lamp Haze): transparent under a fluorescent light, but partially turbid under a zirconium lamp.
L.H(Lamp Haze): transparent under a fluorescent light, but turbid under a zirconium lamp.
V.H(Visual Haze): turbid both under a fluorescent lamp and a zirconium lamp

**[Table 1]**

| | Highest temperature in purification step (°C) | Residence time in purification step (hr) | XDI purity* Immediately after preparation | XDI purity* after 6 months | Whitening of XDI after 6 months | Content of white substances (wt%) | Transparency of lens |
|---|---|---|---|---|---|---|---|
| Example1 | 150 | 15 | 99.60 | 98.60 | × | 0 | C |
| Example2 | 140 | 16 | 99.43 | 98.47 | × | 0 | C |
| Comparat ive | 140 | 32 | 99.52 | 98.04 | ○ | 1.2 | V.H |
| Example1 | | | | | | | |
| Comparative Example2 | 150 | 24 | 99.03 | 98.24 | ○ | 0.8 | S.H |
| Comparative Example3 | 150 | 32 | 99.50 | 98.21 | ○ | 1.4 | V.H |
| Comparative Example4 | 170 | 16 | 99.41 | 87.9 | ○ | 4 | V.H |

| | | | | | | | |
|---|---|---|---|---|---|---|---|
| * area % in GC analysis | | | | | | | |

Referring to Table 1, it can be confirmed that in the case of XDI' s of Examples 1 and 2 prepared by controlling the highest temperature of the purification step to be less than 170 °C, and the residence time in the purification to be 16 hours or less, the purity of XDI maintained excellent, and long-term storage stability was excellent, and thus, whitening did not occur. It was also confirmed that optical devices prepared using the XDI' s of Examples 1 and 2 exhibited excellent transparency.

However, it can be confirmed that in case, during the preparation of XDI, the temperature of the purification step is too high, or the temperature is appropriate but the residence time in the purification step is too long, as in Comparative Examples 1 to 4, although purity immediately after purification is excellent, whitening occurs or the purity significantly decreases, thus deteriorating the stability of XDI, and ultimately, affecting the quality of optical devices prepared using the same.

## Claims

1. A method for preparing diisocyanate comprising:
a reaction step in which diamine or a salt thereof is reacted with phosgene to obtain a reaction mixture; and
a purification step in which diisocyanate is separated from the reaction mixture,
wherein the purification step is conducted at a temperature less than 170 °C for 16 hours or less,
wherein the purification step comprises steps of:
conducting vacuum distillation of the reaction mixture under a first temperature and a first pressure to remove solvents;
conducting vacuum distillation under a second temperature and a second pressure to remove low-boiling impurities; and
conducting thin film distillation under a third temperature and a third pressure to remove oligomers,
wherein the third temperature is equal to or lower than the first temperature and/or the second temperature, and
the third pressure is equal to or lower than the first pressure and/or the second pressure.

2. The method for preparing diisocyanate according to claim 1, wherein the diamine is one or more selected from the group consisting of 1,2-xylylene diamine, 1,3-xylylene diamine, 1,4-xylylene diamine, 1,2-bis(aminomethyl)cyclohexane, 1,3-bis(aminomethyl)cyclohexane, and 1,4-bis(aminomethyl)cyclohexane, and
the diamine salt is one or more selected from the group consisting of 1,2-xylylene diamine hydrochloride, 1,3-xylylene diamine hydrochloride, 1,4-xylylene diamine hydrochloride, 1,2-bis(aminomethyl)cyclohexane hydrochloride, 1,3-bis(aminomethyl)cyclohexane hydrochloride, 1,4-bis(aminomethyl)cyclohexane hydrochloride, 1,2-xylylene diamine carbonate, 1,3-xylylene diamine carbonate, 1,4-xylylene diamine carbonate, 1,2-bis(aminomethyl)cyclohexane carbonate, 1,3-bis(aminomethyl)cyclohexane carbonate, and 1,4-bis(aminomethyl)cyclohexane carbonate.

3. The method for preparing diisocyanate according to claim 1, wherein the reaction step is conducted at a temperature of 80 °C to 180 °C.

4. The method for preparing diisocyanate according to claim 1, wherein the purification step is conducted at a temperature of 100 °C to 150 °C for 5 to 15 hours.

5. The method for preparing diisocyanate according to claim 1, wherein the purification step is conducted under pressure of 0.001 to 50 kPa by vacuum distillation and/or thin film distillation.

6. The method for preparing diisocyanate according to claim 1, wherein the purity of diisocyanate after the purification step is 99% to 100%.

## Patentansprüche

1. Verfahren zur Herstellung eines Diisocyanats, umfassend:
einen Reaktionsschritt, in dem ein Diamin oder ein Salz davon mit Phosgen umgesetzt wird, um ein Reaktionsgemisch zu erhalten; und
einen Aufreinigungsschritt, in dem das Diisocyanat von dem Reaktionsgemisch abgetrennt wird,
wobei der Aufreinigungsschritt bei einer Temperatur von weniger als 170°C für 16 Stunden oder weniger durchgeführt wird,
wobei der Aufreinigungsschritt folgende Schritte umfasst:
Durchführen einer Vakuumdestillation des Reaktionsgemischs bei einer ersten Temperatur und einem ersten Druck zur Entfernung von Lösungsmitteln;
Durchführen einer Vakuumdestillation bei einer zweiten Temperatur und einem zweiten Druck, um niedrigsiedende Verunreinigungen zu entfernen; und
Durchführen einer Dünnschichtdestillation bei einer dritten Temperatur und einem dritten Druck, um Oligomere zu entfernen,
wobei die dritte Temperatur gleich oder niedriger als die erste Temperatur und/oder die zweite Temperatur ist, und
der dritte Druck gleich oder niedriger als der erste Druck und/oder der zweite Druck ist.

2. Verfahren zur Herstellung eines Diisocyanats nach Anspruch 1, wobei das Diamin eines oder mehrere, ausgewählt die aus der Gruppe, bestehend aus 1,2-Xylylendiamin, 1,3-Xylylendiamin, 1,4-Xylylendiamin, 1,2-Bis(aminomethyl)cyclohexan, 1,3-Bis(aminomethyl)cyclohexan und 1,4-Bis(aminomethyl)cyclohexan, ist, und
das Diaminsalz eines oder mehrere, ausgewählt aus der Gruppe, bestehend aus 1,2-Xylylendiaminhydrochlorid, 1,3-Xylylendiaminhydrochlorid, 1,4-Xylylendiaminhydrochlorid, 1,2-Bis(aminomethyl)cyclohexanhydrochlorid, 1,3-Bis(aminomethyl)cyclohexanhydrochlorid, 1,4-Bis(aminomethyl)cyclohexanhydrochlorid, 1,2-Xylylendiamincarbonat, 1,3-Xylylendiamincarbonat, 1,4-Xylylendiamincarbonat, 1,2-Bis(aminomethyl)cyclohexancarbonat, 1,3-Bis(aminomethyl)cyclohexancarbonat und 1,4-Bis(aminomethyl)cyclohexancarbonat, ist.

3. Verfahren zur Herstellung eines Diisocyanats nach Anspruch 1, wobei der Reaktionsschritt bei einer Temperatur von 80 °C bis 180 °C durchgeführt wird.

4. Verfahren zur Herstellung eines Diisocyanats nach Anspruch 1, wobei der Aufreinigungsschritt bei einer Temperatur von 100 °C bis 150 °C für 5 bis 15 Stunden durchgeführt wird.

5. Verfahren zur Herstellung eines Diisocyanats nach Anspruch 1, wobei der Aufreinigungsschritt durch Vakuumdestillation und/oder Dünnschichtdestillation bei einem Druck von 0,001 bis 50 kPa durchgeführt wird.

6. Verfahren zur Herstellung eines Diisocyanats nach Anspruch 1, wobei die Reinheit des Diisocyanats nach dem Aufreinigungsschritt 99% bis 100% beträgt.

## Revendications

1. Procédé de préparation de diisocyanate comprenant :
une étape de réaction consistant à faire réagir une diamine ou un sel de celle-ci avec du phosgène pour obtenir un mélange réactionnel ; et
une étape de purification dans laquelle le diisocyanate est séparé du mélange réactionnel,
dans lequel l'étape de purification est mise en œuvre à une température inférieure à 170 °C pendant 16 heures ou moins,
dans lequel l'étape de purification comprend les étapes consistant à :
effectuer une distillation sous vide du mélange réactionnel à une première température et sous une première pression pour éliminer des solvants ;
effectuer une distillation sous vide à une deuxième température et sous une deuxième pression pour éliminer des impuretés à bas point d'ébullition ; et
effectuer une distillation en film mince à une troisième température et sous une troisième pression pour éliminer des oligomères,
dans lequel la troisième température est égale ou inférieure à la première température et/ou à la deuxième température, et
la troisième pression est égale ou inférieure à la première pression et/ou à la deuxième pression.

2. Procédé de préparation de diisocyanate selon la revendication 1, dans lequel la diamine est une ou plusieurs diamines sélectionnées dans le groupe constitué par la 1,2-xylylènediamine, la 1,3-xylylènediamine, la 1,4-xylylènediamine, le 1,2-bis(aminométhyl)cyclohexane, le 1,3-bis(aminométhyl)cyclohexane et le 1,4-bis(aminométhyl)cyclohexane, et
le sel de diamine est un ou plusieurs sels choisis dans le groupe constitué par le chlorhydrate de 1,2-xylylènediamine, le chlorhydrate de 1,3-xylylènediamine, le chlorhydrate de 1,4-xylylènediamine, le chlorhydrate de 1,2-bis(aminométhyl)cyclohexane, le chlorhydrate de 1,3-bis(aminométhyl)cyclohexane, le chlorhydrate de 1,4-bis(aminométhyl)cyclohexane, le carbonate de 1,2-xylylènediamine, le carbonate de 1,3-xylylènediamine, le carbonate de 1,4-xylylènediamine, le carbonate de 1,2-bis(aminométhyl)cyclohexane, le carbonate de 1,3-bis(aminométhyl)cyclohexane et le carbonate de 1,4-bis(aminométhyl)cyclohexane.

3. Procédé de préparation de diisocyanate selon la revendication 1, dans lequel l'étape de réaction est mise en œuvre à une température de 80 °C à 180 °C.

4. Procédé de préparation de diisocyanate selon la revendication 1, dans lequel l'étape de purification est mise en œuvre à une température de 100 °C à 150 °C pendant 5 à 15 heures.

5. Procédé de préparation de diisocyanate selon la revendication 1, dans lequel l'étape de purification est mise en œuvre sous une pression de 0,001 à 50 kPa par distillation sous vide et/ou distillation en film mince.

6. Procédé de préparation de diisocyanate selon la revendication 1, dans lequel la pureté du diisocyanate après l'étape de purification est de 99 % à 100 %.
